# EUROPEAN PATENT APPLICATION

(11) **EP 4 752 218 A1**
(43) Date of publication of application: **03.06.2026**
(21) Application number: 24216509.0
(22) Date of filing: 29.11.2024
(51) Int. Cl.: C12N 9/10, A23L 33/125, C12N 15/70, C12P 19/00, C12P 19/04

(54) **MUTANT GALACTOSIDASE PERMEASE AND USES THEREOF**

(71) Applicant: OligoScience Biotechnology GmbH, 59199 Bönen (DE)
(72) Inventor: Peracha, Max, 59199 Bönen (DE); Wemhoff, Sabrina, 59199 Bönen (DE); Horvath, Mihaly, 59199 Bönen (DE)
(74) Representative: WSL Patentanwälte Partnerschaft mbB

(57) **Abstract**

The present invention relates to novel mutant galactoside permeases. In particular, the present invention relates to a novel mutant galactoside permease having at least one mutation, which provides a reduced or a lack of proton symporter activity. The present disclosure further relates to a nucleic acid sequence encoding the mutant galactoside permease and a vector comprising the nucleic acid sequence. The present disclosure also relates to a (non-human) host cell and to the advantages of use of said (non-human) host cells for the production of (human milk) oligosaccharides. The present invention further relates to a process for the production of (human milk) oligosaccharides.

## Description

### TECHNICAL FIELD

The present invention relates to novel mutant galactoside permeases. In particular, the present invention relates to a novel mutant galactoside permease having at least 57% sequence identity to the amino acid sequence of SEQ ID NO: 1 and having at least one mutation, which provides a reduced or a lack of proton symporter activity. The present disclosure further relates to a nucleic acid sequence encoding the mutant galactoside permease and a vector comprising the nucleic acid sequence. The present disclosure also relates to a (non-human) host cell and to the advantages of use of said (non-human) host cells for the production of (human milk) oligosaccharides. The present invention further relates to a process for the production of (human milk) oligosaccharides.

### TECHNICAL BACKGROUND

With regard to nutrition, human breast milk is ideal for infant development. Human breast milk contains various oligosaccharides, known as human milk oligosaccharides (HMOs). Industrial production and commercialization of HMOs is of increasing importance. For the breast-fed child HMOs are indigestible; the relevance of HMOs is due to their unique biological effects. HMOs have become key products for nutrition and therapeutic uses as nutraceutical, antimicrobial, anti-inflammatory and immune-modulatory agents. As a result, the industrial scale production of HMOs associated with high yields at low costs, is desirable.

To date, more than 150 distinct HMOs differing in structure have been identified. HMOs consist of combinations of five monosaccharide components, i.e., D-glucose, D-galactose, N-acetyl-glucosamine, L-fucose and N-acetylneuraminic acid.

As a matter of course, obtaining of HMOs from their natural source is restricted. Thus, large-scale industrial production is to be realized only by artificial synthesis. HMOs can be synthesized *in vitro* by chemical or enzymatic methods, however, with minor efficiency. Highly efficient production can be achieved by fermentative processes wherein oligosaccharides are produced by a host cell. Industrial scale fermentative production of individual HMOs made these prebiotic compounds accessible for nutritional and therapeutic uses, such as supplements for infant formula.

HMOs are modified forms of lactose, such as 2'-fucosyllactose, 3'-fucosyllactose, 3'-sialyllactose, 6'-sialyllactose, etc. (listed under point 22). During the fermentative process for the production of such HMOs, a host cell is typically cultivated in the presence of (i) a carbon source, and (ii) a galactoside (sugar), preferably lactose. Lactose is a naturally occurring common disaccharide composed of galactose and glucose, i.e., β-D-galactopyranosyl-(1→4)-D-glucose.

In fermentative production of oligosaccharides, the phenomenon of lactose toxicity, also called lactose killing is of particular interest, since it inhibits growth of many organisms. Lactose killing was first described in 1961 by Von Hofsten (Von Hofsten, B, Biochim. Biophys. Acta 48, 164-171) in a mutant *E*. *coli* strain. During the many years since then, the phenomenon was studied in more detail and in general it can be defined by saying that lactose killing occurs when the lactose transporter gene is (inducibly or constitutively) overexpressed in the presence of lactose or other galactoside substrates (with variously strong effect).

Overexpression of the lactose transporter can occur even in wild type strains under specifically chosen growth conditions, or in recombinant strains where the lactose transporter gene is artificially overexpressed using molecular genetic methods, as it is usually the case in industrial processes. The natural lactose transporter gene *lacY* (galactoside permease) is part of the lac operon, which is transcriptionally induced by the presence of lactose. Via induction by lactose, expression of the lac operon reaches only about 1/3 of its maximum level, regulated by cellular feedback control. Further increase of expression can be achieved, for example, by growing the cells in a minimal medium maintaining a low concentration of lactose (Dykhuisen D. and Hartl D. Journal of Bacteriology 1978 Sept. 876-882), where competition for the substrate causes genetic changes in the cells. Other artificial ways to increase /acY-expression include deleting the lac-repressor gene *lacl,* or replacing the lac promoter with other, stronger promoters.

When these sensitized cells are exposed to a larger amount of lactose, the increased transport capacity will drive the cells to take up more lactose than they can metabolically process, leading to an imbalance between the energy consumption during transport and the energy regeneration by metabolism. Particularly strong effect arises, when lactose catabolism is genetically blocked, even if an alternative carbon source, other than lactose is available in the medium (as it is common in the related industrial processes). As a consequence of this imbalance the chemical energy of the cells depletes. One manifestation of the cellular energy is in the form of a proton gradient along the cellular membrane, that was named chemiosmotic potential by the British chemist Peter D. Mitchell, who was awarded the Nobel Prize in 1978 for this discovery.

Lactose transport is an active transport process, driven by the energy stored in this membrane potential, whereby one molecule of lactose is transported on the price of co-transporting one proton in a precise 1:1 stoichiometry, which mechanism is thereby also called galactoside:proton symport. When the cells take up too much lactose too fast in an uncontrolled way that depletes the membrane potential as well as the biochemical energy-restoring capacity of the cell leading to an energetic crisis. The cells sense this adverse condition and respond to it by drastically saving energy. This involves rapid growth arrest as well as shutting down protein synthesis in general in a mechanism known as stringent response. These metabolic adjustments severely affect HMO production or any other fermentation process that uses lactose.

Since said phenomenon delays growth and inhibits metabolic processes, which consequently has a negative effect on the production of HMOs when lactose is supplied to the medium as a ready molecular building block, it is desirable to prevent lactose killing and concomitantly increase yields of HMOs during industrial scale production. Patent literature exists which has already attempted to solve the problem, which is shortly reviewed here.

WO 2012 / 112 777 A2 proposes a solution against lactose killing either by severely reducing lactose-uptake or by decoupling the growth phase from the production phase in order to obtain at first sufficient biomass. Subsequent to the growth phase, lactose is added in the second phase in order to produce the specific product. Even though the authors could achieve sufficient biomass with this method, the lactose toxicity is unfortunately not a mere growth arrest, but rather the deeper-lying energy crisis that effectively inhibits metabolic output in the production phase.

EP 3 218 509 B1 provides microorganisms being resistant to lactose killing by altering the expression level of a lactose transporter. More specifically, the expression level of said lactose transporter is reduced, thus, leading to a microorganism that is capable to withstand the lactose challenge but still retains at least 50 % of the lactose influx compared to a wild-type expression cassette of said lactose transporter. However, adverse effects on the growth of bacterial cells are still observed.

WO 2022 / 136 568 A1 takes the fact in consideration, that the lactose transporter also possesses regulatory functions exerted via protein-level interaction, for that it is desirable not to decrease the cellular abundance of the LacY protein. Instead, in contrast to the previous patent that manipulates *lacY* transcription level, this patent attempts to decrease lactose transport capacity by mutations that reduce the specific activity of the transporter enzyme. The authors disclose the expression of mutant LacY lactose permeases, with mutation(s) at position(s) 292, and/or 293, and/or 294 compared to the wild-type LacY in a microbial cell. The authors succeeded to reduce the effect of lactose killing, as well as claiming a modest 20% increase in HMO yield obtained from the microorganisms compared to the wild-type LacY-harboring strain.

In light of the above, the present disclosure particularly addresses this object and provides enhanced yields of HMOs without the adverse effects of lactose killing or without reducing the expression level or activity of the galactoside permease.

### SUMMARY OF THE INVENTION

The present disclosure provides a novel mutant galactoside permease, which is capable of transporting lactose without depleting or even using the membrane potential, and thereby avoids lactose killing, even at high lactose concentrations in association with increased HMO production yields. The problem is solved by subject matter of claims 1, 9, 10, 12, and 13. Embodiments are subject matter of claims 2 to 8, 11, 14, and 15.

In particular, the present disclosure provides:
[1] A mutant of a galactoside permease that possesses lactose transport activity and carries at least one mutation, which provides a reduced or a lack of proton symporter activity, wherein the mutation is in at least at one position which corresponds to a conserved position selected from positions 322, 325, 302, 237, 269, 329, 31, 64, 68, 115, 126, 144, 147, 240, 319, 334, 346, 348, 358, 35, 76, 131, 140, 151, 177, 184, 350, 380, and 400 of SEQ ID NO: 1.
[2] The mutant galactoside permease of [1], which has at least 57%, preferably 80%, more preferably 85%, even more preferably at least 90% sequence identity to the amino acid sequence of SEQ ID NO: 1.
[3] The mutant galactoside permease of [1] or [2], wherein the at least one mutation is at a position that is involved in proton translocation of the (wild-type) galactoside permease according to SEQ ID NO: 1.
[4] The mutant galactoside permease of any one of [1] to [3], wherein the mutant galactoside permease:
   (i) has a modified proton symporter activity, preferably a reduced proton symporter activity, more preferably a lack of proton symporter activity; and/or
   (ii) is not capable of mediating galactoside: proton symport with a 1:1 stoichiometry; and/or
   (iii) has a reduced proton translocation activity, preferably a lack of proton translocation activity, as compared to the wild-type galactoside permease according to SEQ ID NO: 1.
[5] The mutant galactoside permease of any one of [1] to [4], wherein the mutant galactoside permease:
   (a) is capable of substrate binding and/or has no mutation in the substrate-binding site; and/or
   (b) has / maintains substrate specificity for galactosides; and/or
   (c) exhibits / maintains galactoside translocation activity.
[6] The mutant galactoside permease of [5], wherein the mutant galactoside permease does not require (full) proton translocation for the binding of galactoside(s).
[7] The mutant galactoside permease of any one of [1] to [6], wherein the at least one mutation is at a position corresponding to any of positions 237 to 334 of SEQ ID NO: 1, preferably at one or more positions corresponding to positions 322, 325, 302, 237, and 269, of the amino acid sequence of SEQ ID NO:1, preferably at the position corresponding to position 322 of the amino acid sequence of SEQ ID NO:1.
[8] The mutant galactoside permease of any one of [1] to [7], wherein the at least one mutation is at a position in helix IX and/or helix X of the galactoside permease according to amino acid sequence of SEQ ID NO:1;
   preferably, wherein the at least one mutation is at a position in transmembrane helix IX selected from 322, 325, 334, 319, and/or in transmembrane helix X at 302 of the amino acid sequence of SEQ ID NO: 1.
[9] The mutant galactoside permease of any one of [1] to [8], wherein the at least one mutation is at least one amino acid substitution.
[10] The mutant galactoside permease of [9], wherein the at least one amino acid substitution comprises a substitution of
   (i) a basic amino acid residue against a basic amino acid residue; and/or
   (ii) a basic amino acid residue against a polar amino acid residue; and/or
   (iii) a basic amino acid residue against a non-polar amino acid residue; and/or
   (iv) a basic amino acid residue against an acidic amino acid residue; and/or
   (v) an acidic amino acid residue against a non-polar amino acid residue; and/or
   (vi) an acidic amino acid residue against a polar amino acid residue; and/or
   (vii) an acidic amino acid residue against an acidic amino acid residue; and/or
   (viii) an acidic amino acid residue against a basic amino acid residue.
[11] The mutant galactoside permease of [9] or [10], wherein:
   (a) the amino acid substitution at the position corresponding to position 322 of the amino acid sequence of SEQ ID NO: 1 is a substitution of histidine against an amino acid residue selected from any one of Y, Q, C, R, K, N, A, M, F, or D, preferably a substitution of histidine against an amino acid residue selected from any one of Q, C, R, K, or N, more preferably a substitution of histidine against an amino acid residue selected from any one of R or K; and/or
   (b) the amino acid substitution at the position corresponding to position 325 of the amino acid sequence of SEQ ID NO: 1 is a substitution of glutamic acid against an amino acid residue selected from any one of C, D, A, Q, K, S, or H, preferably a substitution of glutamic acid against an amino acid residue selected from any one of C, D, or A, more preferably a substitution of glutamic acid against an amino acid residue selected from any one of C or A; and/or
   (c) the amino acid substitution at the position corresponding to position 302 of the amino acid sequence of SEQ ID NO: 1 is a substitution of arginine against an amino acid residue selected from any one of K, H, L, A, S, or C, preferably a substitution of arginine against an amino acid residue selected from any one of K, H, L, A, or S, more preferably a substitution of arginine against an amino acid residue selected from any one of K or H; and/or
   (d) the amino acid substitution at the position corresponding to position 269 of the amino acid sequence of SEQ ID NO: 1 is a substitution of glutamic acid against aspartic acid; and/or
   (e) the amino acid substitution at the position corresponding to position 237 of the amino acid sequence of SEQ ID NO: 1 is a substitution of aspartic acid against cysteine.
[12] The mutant galactoside permease of [11], wherein the mutant galactoside permease comprises at least one of the following amino acid substitutions:
   (i) H322R at the position corresponding to position 322 of the amino acid sequence of SEQ ID NO: 1; and/or
   (ii) E325A at the position corresponding to position 325 of the amino acid sequence of SEQ ID NO: 1; and/or
   (iii) R302K at the position corresponding to position 302 of the amino acid sequence of SEQ ID NO: 1; and/or
   (iv) E269D at the position corresponding to position 269 of the amino acid sequence of SEQ ID NO: 1; and/or
   (v) D237C at the position corresponding to position 237 of the amino acid sequence of SEQ ID NO: 1.
[13] The mutant galactoside permease of any one of [1] to [12], wherein the mutant galactoside permease is a mutant β-galactoside permease, preferably a mutant lactose permease.
[14] A nucleic acid sequence encoding the mutant galactoside permease of any one of [1] to [13].
[15] A vector comprising the nucleic acid sequence of [14].
[16] A (non-human) host cell, preferably a microbial host cell, comprising the nucleic acid sequence of [14], or the vector of [15].
[17] The (non-human) host cell of [16], which is a bacterial cell or a yeast cell.
[18] The (non-human) host cell of [16] or [17], wherein the host cell comprises an exogenous nucleic acid sequence encoding a glycosyltransferase, preferably a fucosyltransferase.
[19] Use of the mutant galactoside permease of any one of [1] to [13], or the nucleic acid sequence of [14], or the vector of [15], or the (non-human) host cell of any one of [16] to [18], for the production of (human milk) oligosaccharides.
[20] A process for the production of (human milk) oligosaccharide, comprising:
   (a) culturing (non-human) host cell of any one of [16] to [18] in the presence of (i) an exogenous carbon source and (ii) a galactoside (sugar), preferably lactose, and under conditions that allow the host cell to produce a (human milk) oligosaccharide, and
   (b) obtaining the (human milk) oligosaccharide from the culture medium and/or the (non-human) host cell.
[21] The process of [20], wherein the process is a (fed) batch or a continuous process.
[22] The process of [20] or [21], wherein the oligosaccharide is selected from the group consisting of 2'-fucosyllactose (2'-FL), 3'-fucosyllactose (3'-FL), 2', 3-difucosyllactose (DFL), lacto-*N*-triose II, lacto-*N*-tetraose (LNT), lacto-*N*-*neo*- tetraose (LNnT), lacto-*N*-fucopentaose I (LNFP-I), lacto-N-neofucopentaose I (LN*n*FP-I), lacto-*N-*fucopentaose II (LNFP-II), lacto-*N*-fucopentaose III (LNFP- III), lacto-*N*-fucopentaose V (LNFP-V), lacto-*N*-neofucopentaose V (LNnFP-V), lacto-*N*-hexaose (LNH), lacto-*N*-neohexaose (LNnH), para-lacto-N-hexaose (paraLNH), para-lacto-N-neohexaose (paraLNnH), difucosyl-lacto-N-neohexaose (DF-LNnH), lacto-*N*-difucosylhexaose I, lacto-*N*-difucosylhexaose II, *para-* lacto-*N*-fucosylhexaose (paraLNH), fucosyl-lacto-*N*-sialylpentaose a (F-LST- a), fucosyl-lacto-*N*-sialylpentaose b (F-LST-b), fucosyl-lacto-*N*-sialylpentaose c (F-LST-c), fucosyl-lacto-*N*-sialylpentaose c, disialyl-lacto-*N-*fucopentaose, 3-fucosyl-3'-sialyllactose (3F-3'-SL), 3-fucosyl-6'-sialyllactose (3F-6'-SL), lacto-*N*-neodifucohexaose I, 3'-sialyllactose (3-SL), 6'-sialyllactose (6-SL), sialyl-lacto-/V-tetraose a (LST-a), sialyllacto-*N*-tetraose b (LST-b), sialyllacto-*N*-tetraose c (LST-c), disialyllacto-*N*-tetraose (DS-LNT), Disialyl-lacto-*N*-fucopentaose (DS-LNFP V), lacto-N-neodifucohxaose I (LNnDFH I), 3'-galactosyllactose (3'- GL), 6'-galactosyllactose (6'-GL), or derivatives thereof.

### BRIEF DESCRIPTION OF THE DRAWINGS

- **Fig. 1**:: Representation of the amino acid sequence of *Escherichia coli* strain BL21 and *Escherichia coli* strain K12, substrain MG 1655, galactoside permease LacY, i.e., SEQ ID NO: 1 as in the attached sequence list.
- **Fig. 2**:: Representation of the nucleic acid sequence of *Escherichia coli* strain BL21 and *Escherichia coli* strain K12 galactoside permease LacY, i.e., SEQ ID NO: 2 as in the attached sequence list.

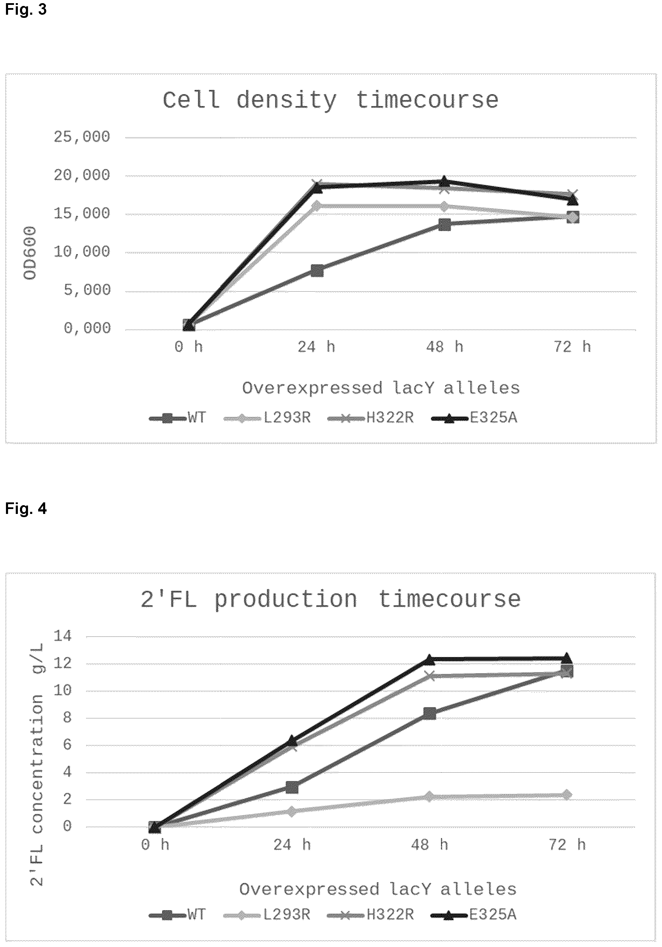

- **Fig. 3**:: Graph illustrating the growth kinetics (OD600) over time (h) of bacterial cells expressing *Escherichia coli* wild-type lactose permease LacY, control mutant LacY (L293R) and proton symport-deficient mutants LacY (H322R) and LacY (E325A).
- **Fig. 4**:: Graph illustrating 2'FL-productivity (mg/L) over time (h) of bacterial strains expressing *Escherichia coli* wild-type lactose permease LacY, control mutant LacY (L293R), mutant LacY (H322R), and mutant LacY (E325A).

### DETAILED DESCRIPTION

The present inventors developed novel mutant galactoside permeases according to the disclosure with unexpected advantageous properties. In particular, it was found that the (over-)expressed mutant galactoside permeases exhibit enhanced production of oligosaccharides, specifically HMOs.

The most economic processes of producing HMO sugars utilize externally added lactose to the fermentation broth as a ready molecular building block. This method, however, poses a toxic effect on the production cells. Surprisingly, the mutant galactoside permeases according to the present invention can advantageously be used in the production of oligosaccharides since host cells according to the invention, that possess any of the mutant galactoside permease alleles are insensitive or much less sensitive to lactose killing, even when the permease is overexpressed. The use of the mutant galactoside permease variants of the present invention, preferably when combined with the overexpression of their genes, results in increased HMO levels compared to control cells. These novel features could not have been expected.

The toxic effect of lactose increases with medium lactose concentration of up to about 5 g/L, where it reaches maximum toxicity with full growth arrest in shake flasks (in fermenter the dynamics is somewhat different). Surprisingly, cells comprising a galactoside permease according to the invention, as well as strains that (over-)express a mutant *lacY* allele according to the present invention tolerate very high medium lactose concentrations, e.g., of up to 100 g/L, without any noticeable negative effect. This is very good for two reasons. Firstly, lactose from concentrated solutions used for fed batch procedure is prone to precipitate in the feeding tube, frequently leading to clogging, therefore the preferred way to add lactose to the fermentation broth is in batches, whereby lactose concentration can temporarily reach higher levels, which the cells must then tolerate. Secondly, since the galactoside permease of the present invention reduce or inactivate the proton symport function of the lactose permease, they also abolish its active transport capability, turning it into a passive carrier. Consequently, the ability of the transporter, to accumulate lactose in the host cells to higher concentrations than in the medium is lost. Since substrate concentration is an important factor for all enzyme reactions, lowered cytoplasmic lactose levels might negatively influence HMO production. By passive carriers, the force that drives transport is the concentration gradient, in other words the transport process is running as long as the medium concentration of the transported substance is higher than that of the cytoplasm. Therefore, in order to support rapid lactose uptake, as well as to reach high intracellular lactose concentrations, the medium lactose concentration must be high, when using the galactoside permease of the present invention, and this is fully applicable since toxicity has been abolished. As the actual experimental data support, according to the present invention, production of HMOs, such as 2'-fucosyllactose (2'-FL), is increased in bacteria (over-)expressing the mutant galactoside permease.

Due to the improved lactose tolerance achieved by the mutant galactoside permease according to the present invention any priming, pretreatment or accommodating the bacteria (over-)expressing said mutant galactoside permease to high lactose conditions, e.g., by cultivation in pre-culture medium with a reduced lactose content, is not required. Further, monitoring / controlling of the lactose content in the culture medium is not required during the fermentative production process of HMOs. Resultantly, the present invention simplifies and improves fermentation protocols for the production of HMOs.

In a first aspect, the present invention refers to mutant galactoside permeases comprising amino acid sequences having at least 57% sequence identity to the amino acid sequence of SEQ ID NO: 1 and having at least one mutation, which provides a reduced or a lack of proton symporter activity.

Galactoside permease, also known as lactose permease, is a transmembrane protein encoded by the *lacY* gene of the *lac* operon in *E. coli* (but it has homologs in many organisms), which facilitates the passage of lactose across the cell membrane. Lactose permease, i.e., LacY, belongs to the family of the Major Facilitators, i.e., membrane proteins, such as uniporters, symporters, and antiporters, that facilitate the influx and efflux of specific small molecules. The transport mechanism is a symport that utilizes the inward directed electrochemical proton gradient across the cell membrane as its driving force for co-translocation of β-galactosides and protons into the cell. LacY can transport lactose, melibiose, lactulose and the analogue methyl-1-thio-β,D-galactopyranoside (TMG), but no sucrose or fructose. Structurally, the majority of known secondary transporters (i.e. co-transporting a solute with another solute, usually an ion) are all related and can be classified into just two common folds (LacY-class and LeuT class) for one of which the archetype is the lactose permease. Despite significant divergence in their primary sequence, they show a high degree of conservation in their overall structure and at specific positions in their sequence. The currently known most divergent galactoside-permease to *E. coli* LacY has been found in *Enterobacter cloacae,* with just 57% sequence identity, annotated as MelY as melibiose permease, is still a functional lactose transporter.

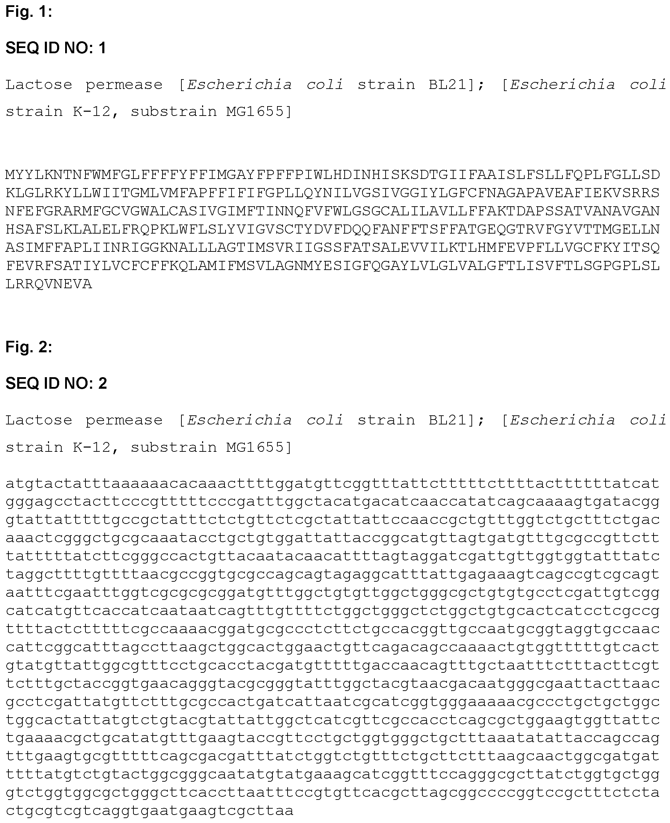

The amino acid sequence of wild-type LacY as represented by SEQ ID NO: 1, refers to the amino acid sequence of LacY of *E. coli* strain BL21 (NCBI accession No: CP010816.1, locus: AJH09253.1) and *E. coli* strain K12 (NCBI accession No: CP025268.1, locus: AUG15133.1 UniProtKB - P02920, last release date on May 29, 2024), shown in Figure 1. The *E. coli* LacY consists of an amino acid chain of 417 amino acid residues in length, and has a calculated mass of 46,503 Da. The secondary structure of the *E. coli* wild-type LacY consists of 12 transmembrane helices organized into two bundles of six helices connected by a long flexible loop. A central hydrophilic cavity contains the lactose binding site and the amino acid residues involved in proton translocation. A more detailed topology of *E*. *coli* wild-type LacY is provided in Table 1.

**Table 1: Topology of E. coli wild-type LacY corresponding to SEQ ID NO: 1.**

| **Amino acid position corresponding to amino acid sequence of SEQ ID NO: 1** | **Domain section** |
|---|---|
| 1 - 7 | cytoplasmic amino-terminal end |
| 8 - 34 | transmembrane helix I |
| 35 - 41 | periplasmic loop P1 |
| 42 - 70 | transmembrane helix II |
| 71 - 74 | cytoplasmic loop C2 |
| 75 - 100 | transmembrane helix III |
| 101 - 104 | periplasmic loop P3 |
| 105 - 129 | transmembrane helix IV |
| 130 - 140 | cytoplasmic loop C4 |
| 141 - 163 | transmembrane helix V |
| 164 - 166 | periplasmic loop P5 |
| 167 - 186 | transmembrane helix VI |
| 187 - 220 | cytoplasmic loop C6 |
| 221 - 249 | transmembrane helix VII |
| 250 - 253 | periplasmic loop P7 |
| 254 - 278 | transmembrane helix VIII |
| 279 - 288 | cytoplasmic loop C8 |
| 289 - 308 | transmembrane helix IX |
| 309 - 311 | periplasmic loop P9 |
| 312 - 334 | transmembrane helix X |
| 335 - 346 | cytoplasmic loop C10 |
| 347 - 374 | transmembrane helix XI |
| 375 - 377 | periplasmic loop P11 |
| 378 - 398 | transmembrane helix XII |
| 399 - 417 | cytoplasmic carboxy-terminal end |

Some amino acid residues play an essential role in the transport of lactose through the protein, such as provided in Table 2.

**Table 2: Essential amino acid residues corresponding to the positions of the amino acid sequence of SEQ ID NO: 1.**

| **Amino acid position corresponding to amino acid sequence of SEQ ID NO: 1** | **Domain function** | **Amino acid residue** |
|---|---|---|
| 126 | substrate binding | E |
| 144 | substrate binding | R |
| 269 | substrate binding/proton translocation | E |
| 302 | proton translocation | R |
| 322 | proton translocation | H |
| 325 | proton translocation | E |

Further, mutagenic analysis of *E. coli* LacY revealed that certain amino acid residues are also relevant for LacY activity. D237 is ion-paired with K358 and they are known to play a structural role. For example, D237 carries a + charge, K358 carries a - charge. If the charges are swapped by a double mutation, or both of them are simultaneously mutated to hydrophobic amino acids, active transport is retained. However, loss of transport activity is created, when one of D237 and K358 remains charged while the other is mutated into a non-charged amino acid. A replacement of the leucine residues at the positions 292, 293, and/or 294 corresponding to the amino acid sequence of SEQ ID NO: 1, with a basic amino acid residue selected from the group consisting of R, K and H, leads to a reduced transport activity for lactose as compared to the *E. coli* wild-type LacY, as provided in Table 3.

**Table 3: Mutagenic results on transport activity of E. coli LacY.**

| **Mutation corresponding to amino acid position of SEQ ID NO: 1** | **Effect** |
|---|---|
| D237N | loss of activity |
| L292R | reduced lactose transport activity |
| L292K | reduced lactose transport activity |
| L292H | reduced lactose transport activity |
| L293R | reduced lactose transport activity |
| L293K | reduced lactose transport activity |
| L293H | reduced lactose transport activity |
| L294R | reduced lactose transport activity |
| L294K | reduced lactose transport activity |
| L294H | reduced lactose transport activity |
| K358T | loss of activity |
| V367A | increase of melibiose transport and impairment of TMG (methyl-1-thio-beta,d-galactopyranoside) transport in *Enterobacter cloacae* |

In various embodiments, the mutant galactoside permease of the present disclosure, has at least 57%, at least 65%, at least 70%, at least 80%, preferably 85%, more preferably at least 90% sequence identity to the amino acid sequence of SEQ ID NO: 1. In additional embodiments, the mutant galactoside permease has at least 91%, at least 92%, at least 93%, at least 94%, at least 95%, at least 96%, at least 97%, at least 98%, or at least 99% sequence identity to the amino acid sequence of SEQ ID NO: 1.

In additional and/or alternative embodiments, the mutant galactoside permease as disclosed herein, has the at least one mutation at a position that is involved in proton translocation of the galactoside permease according to SEQ ID NO: 1.

In particular embodiments, the mutant galactoside permease:
(i) has a modified proton symporter activity, preferably a reduced proton symporter activity, more preferably a lack of proton symporter activity; and/or
(ii) is not capable of mediating galactoside: proton symport with a 1:1 stoichiometry; and/or
(iii) has a reduced proton translocation activity, preferably a lack of proton translocation activity, as comparted to the wild-type galactoside permease according to SEQ ID NO: 1.

In various embodiments, the mutant galactoside permease displays an altered membrane topology as compared to the membrane topology of the wild-type galactoside permease according to SEQ ID NO: 1. Preferably mutations in the transmembrane helix IX and/or X may lead to a decoupled galactoside:proton symport activity. Alternatively, mutations in the transmembrane helix VIII and/or VII may lead to a decoupled galactoside:proton symport activity.

Without being bound by theory, it is assumed that the initial proton acceptor is E269, that forms a H-bond with W151. Ligand-binding causes amino acid side chain displacements and shifts E269 to a more hydrophilic environment, allowing its deprotonation to H322 through water transmission. Deprotonated E269 stabilizes with the hydroxyl group at O3 oxygen of the bound galactoside substrate as well as by forming a salt bridge with R144 that also participates in substrate binding. Protonated H322 moves closer to E325 and transfers the proton onto it. Neutralized E325 shifts into a hydrophobic area between the transporter surface and the lipid bilayer of the membrane, and this intramolecular movement seems to be a key mechanistic element that via force transmission by intramolecular linkages drives the transport of the galactoside substrate. The galactoside substrate dissociates on the cytoplasmic side of the membrane leading to further dynamics within the molecule, whereby E325 gets closer to and deprotonated by the R302 base, through which it returns to its initial state and the cycle can start over.

Neutral substitutions at E325, like E325A, are considered to stabilize the transporter in this normally transitory conformation, keeping the transport channel open and allowing free passive exchange of galactosides between the two sides of the membrane, whereas proton symport is blocked. In addition to that, amino acid positions within the LacY amino acid sequence which are involved in proton translocation have been identified. Unexpectedly, (non-human) host cells expressing the galactosidase permease mutants of the present invention, comprising amino acid substitutions at the identified positions, provide for improved tolerance against lactose killing.

In various embodiments, the mutant galactoside permease:
a) is capable of substrate binding and/or has no mutation in the substrate-binding site; and/or
b) has / maintains substrate specificity for galactosides; and/or
c) exhibits / maintains galactoside translocation activity.

In various embodiments, the mutant galactoside permease does not require (full) proton translocation for the binding of galactoside(s). The galactoside according to the present invention is for example lactose.

In various embodiments, the mutant galactoside permease as disclosed herein has at least one mutation at positions corresponding to any of positions 31 to 400 of SEQ ID NO: 1. For example, in various embodiments, the mutant galactoside permease as disclosed herein has the at least one mutation at one or more positions corresponding to any of positions 295 to 334 of SEQ ID NO: 1.

In various embodiments, the mutant galactoside permease as disclosed herein has the at least one mutation at one or more position of Table 4.

**Table 4: Exemplary mutation positions of the mutant galactoside permease as disclosed herein corresponding to the positions of the amino acid sequence of SEQ ID NO: 1.**

| **Mutation corresponding to amino acid position of SEQ ID NO: 1** | **Domain section** |
|---|---|
| 31 | transmembrane helix I |
| 35 | periplasmic loop P1 |
| 64 | transmembrane helix II |
| 68 | transmembrane helix II |
| 76 | transmembrane helix III |
| 115 | transmembrane helix IV |
| 126 | transmembrane helix IV |
| 131 | cytoplasmic loop C4 |
| 140 | cytoplasmic loop C4 |
| 144 | transmembrane helix V |
| 147 | transmembrane helix V |
| 151 | transmembrane helix V |
| 177 | transmembrane helix VI |
| 184 | transmembrane helix VI |
| 237 | transmembrane helix VII |
| 240 | transmembrane helix VII |
| 269 | transmembrane helix VIII |
| 302 | transmembrane helix IX |
| 319 | transmembrane helix X |
| 322 | transmembrane helix X |
| 325 | transmembrane helix X |
| 334 | transmembrane helix X |
| 346 | transmembrane helix XI |
| 348 | transmembrane helix XI |
| 350 | transmembrane helix XI |
| 358 | transmembrane helix XI |
| 380 | transmembrane helix XII |
| 400 | cytoplasmic carboxy-terminal end |

In various embodiments, the mutant galactoside permease as disclosed herein has at least one mutation at a positions corresponding to 322, 325, 302 329, 31, 64, 68, 115, 126, 144, 147, 237, 240, 269, 319, 334, 346, 348, 358, 35, 76, 131, 140, 151, 177, 184, 350, 380, 400, of SEQ ID NO: 1. In various embodiments, the mutant galactoside permease as disclosed herein has the at least one mutation at one or more positions in transmembrane helix IX and/or transmembrane helix X of galactoside permease according to SEQ ID NO: 1. For example, the mutant galactoside permease as disclosed herein has the at least one mutation selected from 322, 325, 319 and 334 in transmembrane helix X corresponding to the galactoside permease according to SEQ ID NO: 1 and/or 302 in transmembrane helix IX corresponding to the galactoside permease according to SEQ ID NO: 1. For example, the mutant galactoside permease as disclosed herein has the at least one mutation selected from 322, 325, 319 and 334 in helix X corresponding to the galactoside permease according to SEQ ID NO: 1.

In various embodiments, the mutant galactoside permease has at least one mutation at one or more positions corresponding to any of positions 322, 325, 302, 269 and/or 237 of the amino acid sequence of SEQ ID NO: 1, preferably at the position corresponding to position 322 of the amino acid sequence of SEQ ID NO: 1.

In various embodiments, the mutant galactoside permease has a mutation at two or more positions corresponding to any of positions 322, 325, 302, 269 and/or 237 of the amino acid sequence of SEQ ID NO: 1. For example, the mutant galactoside permease has at least two mutation at positions corresponding to 322 and 325 of the amino acid sequence of SEQ ID NO: 1. For example, the mutant galactoside permease has at least two mutation at positions corresponding to 322 and 302 of the amino acid sequence of SEQ ID NO: 1. For example, the mutant galactoside permease has at least two mutation at positions corresponding to 325 and 302 of the amino acid sequence of SEQ ID NO: 1. For example, the mutant galactoside permease has at least two mutation at positions corresponding to 322 and 269 of the amino acid sequence of SEQ ID NO: 1. For example, the mutant galactoside permease has at least two mutation at positions corresponding to 322 and 237 of the amino acid sequence of SEQ ID NO: 1. For example, the mutant galactoside permease has at least two mutation at positions corresponding to 325 and 269 of the amino acid sequence of SEQ ID NO: 1. For example, the mutant galactoside permease has at least two mutation at positions corresponding to 325 and 237 of the amino acid sequence of SEQ ID NO: 1. For example, the mutant galactoside permease has at least two mutation at positions corresponding to 302 and 269 of the amino acid sequence of SEQ ID NO: 1. For example, the mutant galactoside permease has at least two mutation at positions corresponding to 302 and 237 of the amino acid sequence of SEQ ID NO: 1. For example, the mutant galactoside permease has at least two mutation at positions corresponding to 237 and 269 of the amino acid sequence of SEQ ID NO: 1.

In various embodiments, the mutant galactoside permease has a mutation at three or more positions corresponding to any of positions 322, 325, 302, 269 and/or 237 of the amino acid sequence of SEQ ID NO: 1. For example, the mutant galactoside permease has at least three mutation at positions corresponding to 322, 325 and 302 of the amino acid sequence of SEQ ID NO: 1. For example, the mutant galactoside permease has at least three mutation at positions corresponding to 322, 325 and 269 of the amino acid sequence of SEQ ID NO: 1. For example, the mutant galactoside permease has at least three mutation at positions corresponding to 322, 325 and 237 of the amino acid sequence of SEQ ID NO: 1. For example, the mutant galactoside permease has at least three mutation at positions corresponding to 302, 325 and 269 of the amino acid sequence of SEQ ID NO: 1. For example, the mutant galactoside permease has at least three mutation at positions corresponding to 302, 325 and 237 of the amino acid sequence of SEQ ID NO: 1. For example, the mutant galactoside permease has at least three mutation at positions corresponding to 302, 322 and 269 of the amino acid sequence of SEQ ID NO: 1. For example, the mutant galactoside permease has at least three mutation at positions corresponding to 302, 322 and 237 of the amino acid sequence of SEQ ID NO: 1. For example, the mutant galactoside permease has at least three mutation at positions corresponding to 322, 269 and 237 of the amino acid sequence of SEQ ID NO: 1. For example, the mutant galactoside permease has at least three mutation at positions corresponding to 325, 269 and 237 of the amino acid sequence of SEQ ID NO: 1. For example, the mutant galactoside permease has at least three mutation at positions corresponding to 302, 269 and 237 of the amino acid sequence of SEQ ID NO: 1.

In various embodiments, the mutant galactoside permease has a mutation at four or more positions corresponding to any of positions 322, 325, 302, 269 and/or 237 of the amino acid sequence of SEQ ID NO: 1. For example, the mutant galactoside permease has at least four mutation at positions corresponding to 322, 325, 302 and 269 of the amino acid sequence of SEQ ID NO: 1. For example, the mutant galactoside permease has at least four mutation at positions corresponding to 322, 325, 302 and 237 of the amino acid sequence of SEQ ID NO: 1. For example, the mutant galactoside permease has at least four mutation at positions corresponding to 322, 325, 269 and 237 of the amino acid sequence of SEQ ID NO: 1. For example, the mutant galactoside permease has at least four mutation at positions corresponding to 322, 302, 269 and 237 of the amino acid sequence of SEQ ID NO: 1. For example, the mutant galactoside permease has at least four mutation at positions corresponding to 325, 302, 269 and 237 of the amino acid sequence of SEQ ID NO: 1.

In various embodiments, the mutant galactoside permease has at least five mutations at the five positions corresponding to any of positions 322, 325, 302, 269 and 237 of the amino acid sequence of SEQ ID NO: 1.

Said positions 322, 325 in transmembrane helix X and/or 302 in transmembrane helix IX are involved in proton translocation of the galactoside permease according to SEQ ID NO: 1, as shown in Tables 1 and 2.

In various embodiments, the at least one mutation is at least one amino acid substitution. For example, the amino acid substitution is a substitution of the native amino acid at the respective position with any other amino acid of the 19 alternative amino acids. For example, the amino acid substitution is selected from the group consisting of A (Ala, alanine), R (Arg, arginine), N (Asn, asparagine), D (Asp, aspartic acid), C (Cys, cysteine), E (Glu, glutamic acid), Q (Gln, glutamine), G (Gly, glycine), H (His, histidine), I (Ile, isoleucine), L (Leu, leucine), K (Lys, lysine), M (Met, methionine), F (Phe, phenylalanine), P (Pro, proline), S (Ser, serine), T (Thr, threonine), W (Trp, tryptophan), Y (Tyr, tyrosine), and V (Val, valine).

In various embodiments, the at least one amino acid substitution comprises a substitution of
(i) a basic amino acid residue against a basic amino acid residue; and/or
(ii) a basic amino acid residue against a polar amino acid residue; and/or
(iii) a basic amino acid residue against a non-polar amino acid residue; and/or
(iv) a basic amino acid residue against an acidic amino acid residue; and/or
(v) an acidic amino acid residue against a non-polar amino acid residue; and/or
(vi) an acidic amino acid residue against a polar amino acid residue; and/or
(vii) an acidic amino acid residue against an acidic amino acid residue; and/or
(viii) an acidic amino acid residue against a basic amino acid residue.

In various embodiments, the basic residue is an amino acid selected from the group consisting of histidine (His, H), arginine (Arg, R), and lysine (Lys, K).

In various embodiments, the polar amino acid residue is an amino acid residue selected from the group consisting of serine (Ser, S), tyrosine (Tyr, Y), asparagine (Asn, N), and glutamine (Gin, Q).

In various embodiments, the non-polar amino acid residue is an amino acid selected from the group consisting of alanine (Ala, A), cysteine (Cys, C), leucine (Leu, L), methionine (Met, M), and phenylalanine (Phe, F).

In various embodiments, the acidic amino acid residue is aspartic acid (Asp, D).

In various embodiments, the amino acid substitution at the position corresponding to position 322 of the amino acid sequence of SEQ ID NO: 1 is a substitution of histidine against an amino acid residue selected from any one of Y, Q, C, R, K, N, A, M, F, or D. In preferred embodiments, the amino acid substitution at the position corresponding to position 322 of the amino acid sequence of SEQ ID NO: 1 is a substitution of histidine against an amino acid residue selected from any one of Q, C, R, K, or N. In more preferred embodiments, the amino acid substitution at the position corresponding to position 322 of the amino acid sequence of SEQ ID NO: 1 is a substitution of histidine against an amino acid residue selected from any one of R or K.

In various embodiments, the amino acid substitution at the position corresponding to position 325 of the amino acid sequence of SEQ ID NO: 1 is a substitution of glutamic acid against an amino acid residue selected from any one of C, D, A, Q, K, S, or H. In preferred embodiments, the amino acid substitution at the position corresponding to position 325 of the amino acid sequence of SEQ ID NO: 1 is a substitution of glutamic acid against an amino acid residue selected from any one of C, D, or A. In more preferred embodiments, the amino acid substitution at the position corresponding to position 325 of the amino acid sequence of SEQ ID NO: 1 is a substitution of glutamic acid against an amino acid residue selected from any one of C or A.

In various embodiments, the amino acid substitution at the position corresponding to position 302 of the amino acid sequence of SEQ ID NO: 1 is a substitution of arginine against an amino acid residue selected from any one of K, H, L, A, S, or C. In preferred embodiments, the amino acid substitution at the position corresponding to position 302 of the amino acid sequence of SEQ ID NO: 1 is a substitution of arginine against an amino acid residue selected from any one of K, H, L, A, or S. In more preferred embodiments, the amino acid substitution at the position corresponding to position 302 of the amino acid sequence of SEQ ID NO: 1 is a substitution of arginine against an amino acid residue selected from any one of K or H.

In various embodiments, the amino acid substitution at the position corresponding to position 269 of the amino acid sequence of SEQ ID NO: 1 is a substitution of glutamic acid against an amino acid residue selected from any one of Q, C, or D. For example, the amino acid substitution at the position corresponding to position 269 of the amino acid sequence of SEQ ID NO: 1 is a substitution of glutamic acid against aspartic acid (D).

In various embodiments, the amino acid substitution at the position corresponding to position 237 of the amino acid sequence of SEQ ID NO: 1 is a substitution of aspartic acid against an amino acid residue selected from any one of C, A, or K. For example, the amino acid substitution at the position corresponding to position 269 of the amino acid sequence of SEQ ID NO: 1 is a substitution of aspartic acid against glutamic acid cysteine (C).

In various embodiments, the amino acid substitution at the position corresponding to position 334 of the amino acid sequence of SEQ ID NO: 1 is a substitution of phenylalanine against cysteine (C).

In various embodiments, the amino acid substitution at the position corresponding to position 319 of the amino acid sequence of SEQ ID NO: 1 is a substitution of lysine against cysteine (C).

In other particularly preferred embodiments, the mutant galactoside permease comprises at least one of the following amino acid substitutions:
(i) H322R at the position corresponding to position 322 of the amino acid sequence of SEQ ID NO: 1; and/or
(ii) E325A at the position corresponding to position 325 of the amino acid sequence of SEQ ID NO: 1; and/or
(iii) R302K at the position corresponding to position 302 of the amino acid sequence of SEQ ID NO: 1; and/or
(iv) E269D at the position corresponding to position 269 of the amino acid sequence of SEQ ID NO: 1; and/or
(v) D237C at the position corresponding to position 237 of the amino acid sequence of SEQ ID NO: 1.

In various embodiments, the mutant galactoside permease is a mutant β-galactoside permease, preferably a mutant lactose permease.

In a second aspect, a nucleic acid sequence encoding the mutant galactoside permease according to the present invention is provided. A nucleic acid sequence encoding the wild-type *lacY* gene is represented by SEQ ID NO: 2. The nucleic acid sequence encoding the mutant galactoside permease is a nucleotide which encodes any one of the mutant galactoside permease described in the present disclosure.

In various embodiments, the nucleic acid sequence encoding the mutant galactoside permease comprising the amino acid substitution at the position 322 corresponding to the amino acid position represented by SEQ ID NO: 1, comprises a codon selected from the group consisting of CGG, CGA, CGC, CGT, AGA, AGG instead of a codon encoding the histidine residue, i.e., CAC or CAT.

In various embodiments, the nucleic acid sequence encoding the mutant galactoside permease comprising the amino acid substitution at the position 325 corresponding to the amino acid position represented by SEQ ID NO: 1, comprises a codon selected from the group consisting of GCT, GCC, GCA, GCG instead of a codon encoding the glutamic acid residue, i.e., GAG or GAA.

In various embodiments, the nucleic acid sequence encoding the mutant galactoside permease comprising the amino acid substitution at the position 302 corresponding to the amino acid position represented by SEQ ID NO: 1, comprises a codon selected from the group consisting of AAG, AAA instead of a codon encoding the arginine residue, i.e., CGG, CGA, CGC, CGT, AGG or AGA.

In various embodiments, the mutant galactoside permease has at least two mutations selected from H322R, E325A, R302K, E269D and/or D237C, wherein the amino acid positions correspond to the amino acid sequence of SEQ ID NO: 1. For example, the mutant galactoside permease has at least the two mutation H322R and E325A, wherein the amino acid positions correspond to the amino acid sequence of SEQ ID NO: 1. For example, the mutant galactoside permease has at least the two mutation H322R and R302K, wherein the amino acid positions correspond to the amino acid sequence of SEQ ID NO: 1. For example, the mutant galactoside permease has at least the two mutation E325A and R302K, wherein the amino acid positions correspond to the amino acid sequence of SEQ ID NO: 1. For example, the mutant galactoside permease has at least the two mutation H322R and E269D, wherein the amino acid positions correspond to the amino acid sequence of SEQ ID NO: 1. For example, the mutant galactoside permease has at least the two mutation H322R and D237C, wherein the amino acid positions correspond to the amino acid sequence of SEQ ID NO: 1. For example, the mutant galactoside permease has at least the two mutation E325A and E269D, wherein the amino acid positions correspond to the amino acid sequence of SEQ ID NO: 1. For example, the mutant galactoside permease has at least the two mutation E325A and D237C, wherein the amino acid positions correspond to the amino acid sequence of SEQ ID NO: 1. For example, the mutant galactoside permease has at least the two mutation R302K and E269D, wherein the amino acid positions correspond to the amino acid sequence of SEQ ID NO: 1. For example, the mutant galactoside permease has at least the two mutation R302K and D237C, wherein the amino acid positions correspond to the amino acid sequence of SEQ ID NO: 1. For example, the mutant galactoside permease has at least the two mutation E269D and D237C, wherein the amino acid positions correspond to the amino acid sequence of SEQ ID NO: 1.

In various embodiments, the mutant galactoside permease has at least three mutations selected from H322R, E325A, R302K, E269D and/or D237C, wherein the amino acid positions correspond to the amino acid sequence of SEQ ID NO: 1. For example, the mutant galactoside permease has at least the three mutation H322R, E325A and R302K, wherein the amino acid positions correspond to the amino acid sequence of SEQ ID NO: 1. For example, the mutant galactoside permease has at least the three mutation H322R, E325A and E269D, wherein the amino acid positions correspond to the amino acid sequence of SEQ ID NO: 1. For example, the mutant galactoside permease has at least the three mutation H322R, E325A and D237C, wherein the amino acid positions correspond to the amino acid sequence of SEQ ID NO: 1. For example, the mutant galactoside permease has at least the three mutation R302K, E325A and E269D, wherein the amino acid positions correspond to the amino acid sequence of SEQ ID NO: 1. For example, the mutant galactoside permease has at least the three mutation R302K, E325A and D237C, wherein the amino acid positions correspond to the amino acid sequence of SEQ ID NO: 1. For example, the mutant galactoside permease has at least the three mutation R302K, H322R and E269D, wherein the amino acid positions correspond to the amino acid sequence of SEQ ID NO: 1. For example, the mutant galactoside permease has at least the three mutation R302K, H322R and D237C, wherein the amino acid positions correspond to the amino acid sequence of SEQ ID NO: 1.For example, the mutant galactoside permease has at least the three mutation H322R, E269D and D237C, wherein the amino acid positions correspond to the amino acid sequence of SEQ ID NO: 1. For example, the mutant galactoside permease has at least the three mutation E325A, E269D and D237C, wherein the amino acid positions correspond to the amino acid sequence of SEQ ID NO: 1. For example, the mutant galactoside permease has at least the three mutation R302K, E269D and D237C, wherein the amino acid positions correspond to the amino acid sequence of SEQ ID NO: 1.

In various embodiments, the mutant galactoside permease has at least four mutations selected from H322R, E325A, R302K, E269D and/or D237C, wherein the amino acid positions correspond to the amino acid sequence of SEQ ID NO: 1. For example, the mutant galactoside permease has at least the four mutation H322R, E325A, R302K and E269D, wherein the amino acid positions correspond to the amino acid sequence of SEQ ID NO: 1. For example, the mutant galactoside permease has at least the four mutation H322R, E325A, R302K and D237C, wherein the amino acid positions correspond to the amino acid sequence of SEQ ID NO: 1. For example, the mutant galactoside permease has at least the four mutation H322R, E325A, E269D and D237C, wherein the amino acid positions correspond to the amino acid sequence of SEQ ID NO: 1. For example, the mutant galactoside permease has at least the four mutation H322R, R302K, E269D and D237C, wherein the amino acid positions correspond to the amino acid sequence of SEQ ID NO: 1. For example, the mutant galactoside permease has at least the four mutation E325A, R302K, E269D and D237C, wherein the amino acid positions correspond to the amino acid sequence of SEQ ID NO: 1.

In various embodiments, the mutant galactoside permease has at least five mutations selected from H322R, E325A, R302K, E269D and D237C, wherein the amino acid positions correspond to the amino acid sequence of SEQ ID NO: 1.

In additional embodiments, the nucleic acid sequence which encodes the mutant galactoside permease according to the present disclosure, has a sequence identity of at least 45%, at least 50%, at least 55%, at least 60%, at least 65%, at least 70%, at least 75%, at least 80%, at least 85%, at least 90%, at least 95%, at least 98%, or at least 99% to the nucleotide sequence of SEQ ID NO: 2.

In various embodiments, the nucleic acid sequence encoding the mutant galactoside permease according to the invention, is stably integrated into the genomic DNA of the host cell.

The term "encodes" and "codes for" refers broadly to any process whereby the information in a polymeric macromolecule is used to direct the production of a second molecule that is different from the first. The second molecule may have a chemical structure that is different from the chemical nature of the first molecule. For example, in some aspects, the term "encode" describes the process of semi-conservative DNA replication, where one strand of a double-stranded DNA molecule is used as a template to encode a newly synthesized complementary sister strand by a DNA-dependent DNA polymerase. In other aspects, a DNA molecule can encode an RNA molecule (e.g., by the process of transcription that uses a DNA-dependent RNA polymerase enzyme). Also, an RNA molecule can encode a polynucleotide, as in the process of translation. When used to describe the process of translation, the term "encode" also extends to the triplet codon that encodes an amino acid. In some aspects, an RNA molecule can encode a DNA molecule, e.g., by the process of reverse transcription incorporating an RNA-dependent DNA polymerase. In another aspect, a DNA molecule can encode a polypeptide, where it is understood that "encode" as used in that case incorporates both the processes of transcription and translation.

The terms "nucleic acid", "nucleotide", and "polynucleotide" as used herein are used interchangeably and refer to a single or double-stranded polymer of deoxyribonucleotide bases or ribonucleotide bases read from the 5' to the 3' end and include double stranded DNA (dsDNA), single stranded DNA (ssDNA), single stranded RNA (ssRNA), double stranded RNA (dsRNA), genomic DNA, complementary DNA (cDNA), complementary RNA (cRNA), recombinant DNA or recombinant RNA and derivatives thereof, such as those containing modified backbones. Preferably, a polynucleotide, particularly to be stably integrated into the genome of the host cell, is a DNA or cDNA. Polynucleotides according to the invention can be prepared in different ways (e.g. by chemical synthesis, by gene cloning, etc.) and can take various forms (e.g. linear or branched, single or double stranded, or a hybrid thereof, primes, probes etc.) The term "nucleotide sequence" or "nucleic acid sequence" refers to both the sense and antisense strands of a nucleic acid as either individual single strands or in the duplex.

"Nucleic acid sequence", "gene" or "sequences" can be introduced into a target cell directly or preferably by using an "expression vector". Methods used to construct vectors are well known to the person skilled in the art and described in various publications. In particular, techniques for constructing suitable vectors, including a description of functional components such as promoters, enhancers, termination and polyadenylation signals, selection markers, origin of replication, and in case of eukaryotic host cells splicing signals, are reviewed in considerable details in (Sambrook J, et al., 1989. Molecular Cloning: A Laboratory Manual. Cold Spring Harbor: Cold Spring Harbor Laboratory Press) and references cited therein. Vectors may include but are not limited to plasmid vectors, phagemids, cosmids or artificial/mini-chromosomes (e.g. ACE). The eukaryotic expression vectors will typically contain also prokaryotic sequences that facilitate the propagation of the vector in bacteria such as an origin of replication and antibiotic resistance genes for selection in bacteria. A variety of eukaryotic and prokaryotic expression vectors, containing a cloning site into which a polynucleotide can be operably linked, are well known in the art and some are commercially available from companies such as pET (Novagen/Merck Biosciences), pQE (Qiagen), pBAD (Invitrogen/Thermo Fisher Scientific), pF vectors (Promega). Usually, expression vectors also comprise an expression cassette encoding a selectable marker, allowing selection of host cells carrying said expression marker.

The term "stable integration" or "stably integrated" as used in the patent refers to a polynucleotide being introduced into a host cell genome, as opposed to transiently introduced polynucleotides that remain separate from the genomic DNA of the host cell. Stable integration may occur by homologous recombination or other types of recombination. Stable integration may comprise a step of transient introduction of a polynucleotide into a host cell.

According to the third aspect, a vector comprising the nucleic acid sequence according to the present invention is provided.

A "vector" is a nucleic acid that can be used to introduce a polynucleotide into a cell. One type of vector is a "plasmid", which refers to a linear or circular double stranded DNA molecule into which additional nucleic acid sequences can be ligated. Preferably, the vector is a prokaryotic vector or a eukaryotic vector integrating into the genome of a (non-human) host cell and culturing under selective pressure, and thereby are replicated along with the host genome. A vector can be used to direct the expression of a polynucleotide in a cell.

In various embodiments, the vector comprises an expression cassette comprising the nucleic acid sequence according to the invention.

The term "expression" as used herein refers to transcription and/or translation of a nucleic acid sequence within a host cell. The level of expression of a gene product in a host cell may be determined on the basis of either the amount of corresponding RNA that is present in the cell, or the amount of the polypeptide encoded by the selected sequence. For example, RNA transcribed from a selected sequence can be quantified by Northern blot hybridization, ribonuclease RNA protection, *in situ* hybridization to cellular RNA or by PCR, such as qPCR. Proteins encoded by a selected sequence can be quantitated by various methods, e.g. by photometric measurement, by ELISA, by Western Blotting, by radioimmunoassay, by immunoprecipitation, by assaying for the biological activity of the protein.

As used herein, the term "expression cassette" refers to the part of a vector comprising one or more genes encoding for an RNA or a protein and the sequences controlling their expression. Thus, it comprises a promoter sequence, an open reading frame a 3'-untranslated region, typically containing a polyadenylation site. Preferably, the vector is an expression vector comprising one or more gene(s) encoding for the recombinant protein. It may be part of a vector, typically an expression vector including a plasmid or a viral vector. It may also be integrated into the genome by random or targeted integration, such as by homologous recombination. An expression cassette is prepared using cloning techniques and does therefore not refer to a natural occurring gene structure.

A "promoter" or "promoter sequence" is a DNA regulatory region capable of binding RNA polymerase in a cell and initiating transcription of a downstream (3' direction) coding sequence. A promoter sequence is bounded at its 3' terminus by the transcription initiation site and extends upstream in 5' direction. For example, a promoter is about 50 - 3000, about 100 - 2500, about 200 - 2000, about 300 - 1500, or about 350 - 1000 base long. A promoter sequence comprises a transcription initiation site, as well as protein binding domains (consensus sequences) responsible for the binding of the RNA polymerase. Prokaryotic promoters contain a Pribnow box. Eukaryotic promoters often, but not always, contain "TATA" boxes and "CAT" boxes. Promoter sequences often contain additional consensus sequences recognized by proteins involved in regulating expression of the respective gene. Regulation of gene expression by a promoter can occur by enhancing or inhibiting binding of a regulatory protein. Enhancing or inhibiting the binding or a regulatory protein can occur by many different means, including but not limited to, base modifications (i.e., methylation) or protein modification (i.e., phosphorylation). For example, the promoter is a promoter which overexpresses the mutant galactoside permease as disclosed herein. Overexpression as used herein refers to an expression of the mutant galactoside permease as disclosed herein which is higher than the expression of the galactoside permease under the wild-type (native) promoter. Overexpression results in a higher number of transcripts and/or protein of the mutant galactoside permease as disclosed herein compared to expression under the wild-type (native) promoter.

In preferred embodiments, the (non-human) host cell expresses the mutant galactoside permease, wherein the at least one or more mutation is at positions corresponding to positions 322, 325, 302, 269 and/or 237 of the amino acid sequence of SEQ ID NO: 1.

In various embodiments the (non-human) host cell contains a nucleic acid sequence encoding the mutant galactoside permease as disclosed herein. The nucleic acid sequence is for expression of the mutant galactoside permease according to the invention. In various embodiments the nucleotide sequence for expression of the mutant galactoside permease is operably linked to expression control sequences.

In preferred embodiments, the (non-human) host cell is a microbial cell, preferably a bacterial cell or a yeast cell, more preferably a bacterial cell.

The term "organism", "(non-human) host cell" or "cell" as indicated herein refers to a microbial host cell. The microbial cell may be a prokaryotic cell or a eukaryotic cell. Suitable microbial host cells include bacterial cells, yeast cells, archaebacterial cells, and fungal cells.

In preferred embodiments, the prokaryotic cell is a bacterial cell selected from bacteria of a genus selected from the group consisting of *Bacillus, Bifidobacerium, Clostridium, Corynebacterium, Enterococcus, Lactobacillus, Lactococcus, Micrococcus, Micromonospora, Pseudomonas, Rhodococcus,* and *Sporolactobacillus.* In additional and/or alternative embodiments, the bacterial species are *Bacillus subtilis, Bacillus licheniformis, Bacillus coagulans, Bacillus thermophilus, Bacillus laterosporus, Bacillus megaterium, Bacillus mycoides, Bacillus pumilus, Bacillus lentus, Bacillus cereus, Bacillus circulans, Bifidobacterium longum, Bifidobacterium infantis, Bifidobacterium bifidum, Citrobacter freundii, Clostridium cellulolyticum, Clostridium Ijungdahlii, Clostridium autoethanogenum, Clostridium acetobutylicum, Corynebacterium glutamicum, Enterococcus faecium, Entercoccus thermophiles, Escherichia coli, Erwinia herbicola (Pantoea agglomerans), Lactobacillus acidophilus, Lactobacillus salivarius, Lactobacillus plantarum, Lactobacillus helveticus, Lactobacillus delbrueckii, Lactobacillus rhamnosus, Lactobacillus bulgaricus, Lactobacillus crispatus, Lactobacillus gasseri, Lactobacillus casei, Lactobacillus reuteri, Lactobacillus jensenii, Lactobacillus lactis, Pantoea citrea, Pectobacterium carotovorum, Proprionibacterium freudenreichii, Pseudomonas fluorescens, Pseudomonas aeruginosa, Streptococcus thermophiles,* and *Xanthomonas campestris.*

In various embodiments, the eukaryotic cell is a yeast cell, preferably a yeast cell selected from the group consisting of *Saccharomyces sp.,* in particular *Saccharomyces cerevisiae, Saccharomycopsis sp., Pichia sp.,* more preferably *Pichia pastoris, Hansenula sp., Kluyveromyces sp., Yarrowia sp., Rhodotorula sp.,* and *Schizosaccharomyces sp..*

In various embodiments, the (non-human) host cell comprises a nucleic acid sequence encoding a glycosyltransferase, preferably a fucosyltransferase. For example, the host cell comprises a nucleic acid sequence encoding an exogenous glycosyltransferase, preferably a fucosyltransferase. For example, the host cell comprises a nucleic acid sequence encoding an endogenous glycosyltransferase, preferably a fucosyltransferase.

Glycosyltransferases transfer a monosaccharide moiety from a nucleotide activated monosaccharide as donor substrate to an acceptor molecule. The glycosyltransferase may be selected from the group consisting of fucosyltransferases, siayltransferases, mannosyltransferases, N-acetylglucosaminyltransferases, N-acetylgalactosaminyl-transferases, and galactosyltransferases. The acceptor molecule may be selected from the group consisting of lactose and oligosaccharides bearing a lactose moiety at their reducing end.

According to another aspect, use of the mutant galactoside permease or the nucleic acid sequence, or the vector, or the (non-human) host cell according to the present disclosure for the production of (human milk) oligosaccharides is provided.

The term "oligosaccharide" as used herein refers to a saccharide molecule consisting of three to twenty monosaccharide residues, wherein each of said monosaccharide residues is bound to at least one other of said monosaccharide units by a glycosidic linkage. The oligosaccharide may be a linear chain of monosaccharide residues or a branched chain of monosaccharide residues.

In an additional and/or alternative embodiment, the oligosaccharide is a human milk oligosaccharide.

In a further aspect, a process for the production of (human milk) oligosaccharide is provided, comprising:
(a) culturing (non-human) host cell according to the present invention in the presence of (i) an exogenous carbon source and (ii) a galactoside (sugar), preferably lactose, and under conditions that allow the host cell to produce a (human milk) oligosaccharide, and
(b) obtaining the (human milk) oligosaccharide from the culture medium and/or the (non-human) host cell.

For example, a process for the production of (human milk) oligosaccharide according to the present invention does not require a pre-culturing of the (non-human) host cell in a culture medium having reduced content of galactoside (sugar), preferably lactose, in order to prime the (non-human) host cell. For example, the process for the production of (human milk) oligosaccharide according to the present invention does not require a controlled feeding of the galactoside (sugar), preferably lactose, to the culture medium. For example, the process for the production of (human milk) oligosaccharide according to the present invention does not require to monitor/control the content of the galactoside (sugar), preferably lactose, in the culture medium.

In various embodiments, culturing is for example performed without pre-cultivation the of (non-human) host cells in culture medium having a reduced content of galactoside (sugar), preferably lactose. Further, culturing is for example performed without monitoring / controlling the content of the content of the galactoside (sugar), preferably lactose, during the production process.

In various embodiments, culturing is for example performed under conditions having a high content of galactoside (sugar), preferably lactose. For example, culturing is performed in culture medium having a concentration of galactoside (sugar), preferably lactose, of at least 50, at least 60, at least 70, at least 75, at least 80, at least 85, at least 90, at least 95, at least 100, at least 105, at least 110, at least 115, at least 120, at least 125 or at least 130 g/L. For example, culturing is performed in culture medium having a concentration of galactoside (sugar), preferably lactose, concentration of about 20 - 130 g/L, 25 - 110 g/L, 30 - 100 g/L, or 35 - 90 g/L.

The term "exogenous" as used herein refers to any compound, amino acid sequence and/or nucleic acid sequence that has an external origin, (i.e., not derived from the native host-cell). "Exogenous" as used herein may refer to any external origin, such as any compound, amino acid sequence and/or nucleic acid sequence derived from different species, genus, family, order, class, phylum and/or domain. For example, "exogenous" as used herein refers to any compound, amino acid sequence and/or nucleic acid sequence that is added to a (non-human) host cell. For example, "exogenous" as used herein refers to any compound and/or nucleic acid sequence that is added to the culture medium of the (non-human) host cell prior to and/or during cultivation of the (non-human) host cell. Usually, the compound as such is added to the culture medium. The compound can also be produced by a cell that is present in and/or has been added to the culture medium.

The term "endogenous" as used herein refers to any compound, amino acid sequence and/or nucleic acid sequence that has a native origin, (i.e., internally derived from the native host-cell). For example, "endogenous" as used herein refers to any compound, amino acid sequence and/or nucleic acid sequence that is native to the (non-human) host cell, i.e., not added to the (non-human) host cell.

The "medium" or "culture medium" described herein, relates to any solution containing necessary substrates for an organism to grow. These substrates include, but are not limited to, nitrogen sources such as ammonium salts, nitrate salts, yeast extract, pepton, casamino, and/or amino acids, phosphor sources such as but not limited to phosphate salts, elements such as but not limited to copper, cobalt, iron, selenium, iodium, molybdate, magnesium, calcium, potassium, sodium, zinc nickel, manganese, and/or, boric acid and/or vitamins such as but not limited to thiamine, pantothenic acid, and/or niacin and/or an exogenous carbon sources. Exogenous carbon sources comprise, but are not limited to, glycerol, maltose, glucose, fructose, sucrose, fucose, mannose, sialic acid, starch, cellulose, polyols, such as but not limited to mannitol, xylitol, sorbitol, organic acids, such as but not limited to lactate, succinate, acetate, and/or, pentoses, such as but not limited to xylose and arabinose. In preferred embodiments, the exogenous carbon source is glycerol and/or glucose. For example, the exogenous carbon source is glucose.

The term "obtaining" as used herein relates to isolation or extraction of the synthesized oligosaccharides from the host cells in the culture medium. In general, this is performed by methods well known to the person skilled in the art. Typically, cells are harvested by centrifugation followed by subsequent separation of the supernatant from the cell harvest.

In various embodiments, the process is a (fed) batch or a continuous process. The term "fed batch process" relates to an operational technique in biotechnological processes, where one or more substrates are fed (supplied) to the bioreactor during cultivation and in which the products remain in the bioreactor until the end of the feeding run. The concentration of fed-substrate is controlled in the culture medium at arbitrarily desired levels.

The term "continuous process" relates to a flow production method used to produce materials without interruption. Materials, such as dry bulk or fluids are processed continuously in motion, undergoing chemical reactions or subject to mechanical or heat treatment. Continuous processing is contrasted with a fed batch production.

In various embodiments, the process for the production of HMOs is a process for improvement of the efficacy and reliability of producing HMOs in or by the (non-human) host cell, wherein the host cell is cultivated in the presence of exogenous lactose.

In preferred embodiments, the process for production of HMOs is a large-scale and/or industrial production process.

The terms "large-scale", "industrial scale", "industrial" indicate the production of the oligosaccharide using microbial fermentation in a volume of fermentation broth in the full range of 100 L, 500 L, 1000 L, 5000 L, 10,000 L, 50,000 L, 100,000 L or 200,000 L.

In a preferred embodiment the human milk oligosaccharide and/or oligosaccharide is selected from the group consisting of 2'-fucosyllactose (2'-FL), 3-fucosyllactose (3-FL), 2', 3-difucosyllactose (DFL), lacto-*N*-triose II, lacto-*N*-tetraose (LNT), lacto-N-neo-tetraose (LNnT), lacto-*N*-fucopentaose I (LNFP-I), lacto-N-neofucopentaose I (LN*n*FP-I), lacto-*N-*fucopentaose II (LNFP-II), lacto-*N*-fucopentaose III (LNFP-III), lacto-*N*-fucopentaose V (LNFP-V), lacto-*N*-*neo*fucopentaose V (LNnFP-V), lacto-*N*-hexaose (LNH), lacto-*N-*neohexaose (LNnH), para-lacto-N-hexaose (paraLNH), para-lacto-N-neohexaose (paraLNnH), difucosyl-lacto-N-neohexaose (DF-LNnH), lacto-*N*-difucosylhexaose I, lacto-*N-*difucosylhexaose II, *para*-lacto-*N*-fucosylhexaose (paraLNH), fucosyl-lacto-*N*-sialylpentaose a (F-LST- a), fucosyl-lacto-*N*-sialylpentaose b (F-LST-b), fucosyl-lacto-*N*-sialylpentaose c (F-LST-c), disialyl-lacto-*N*-fucopentaose (DS-LNFP), 3-fucosyl-3'-sialyllactose (3F-3'-SL), 3-fucosyl-6'-sialyllactose (3F-6'-SL), lacto-*N*-neodifucohexaose I (LNnDFH I), 3'-sialyllactose (3-SL), 6'-sialyllactose (6-SL), sialyl-lacto-*N*-tetraose a (LST-a), sialyllacto-*N*-tetraose b (LST-b), sialyllacto-*N*-tetraose c (LST-c), disialyllacto-*N*-tetraose (DS-LNT), disialyl-lacto-*N-*fucopentaose V (DS-LNFP V), lacto-N-neodifucohexaose I (LNnDFH I), 3'-galactosyllactose (3'-GL), 6'-galactosyllactose (6'-GL), or derivatives thereof.

The general embodiments "comprising of" or "comprised of" encompass the more specific embodiment "consisting of". Furthermore, singular and plural forms are not used in a limiting way. As used herein, the singular forms "a", "an" and "the" designate both the singular and the plural, unless expressly stated to designate the singular only.

The term "corresponding to the sequence" or "corresponds to the sequence", as used herein includes the defined sequence of *Escherichia coli* having the sequence defined of nucleotides of SEQ ID NO: 2. The skilled person will understand that genomic sequences of microbial cells vary and may therefore not be identical with sequences obtained from LacY of *E. coli* strain BL21 (NCBI accession No: CP010816.1, locus: AJH09253.1) and *E. coli* strain K12 (NCBI accession No: CP025268.1, locus: AUG15133.1) and as shown in SEQ ID NO: 2. However, using sequence alignment, the skilled person would know how to identify the sequence in a specific microbial cell corresponding to the sequence as defined in SEQ ID NO: 1. Such corresponding sequence would have at least 57% identity with the sequence defined in SEQ ID NO: 1, preferably at least 80% identity with the sequence defined in SEQ ID NO: 1 or is identical with SEQ ID NO: 1. The corresponding sequence may also contain recombinant insertions, which is not to be considered for determining the corresponding sequence.

The term "protein" is used interchangeably with "amino acid sequence" or "polypeptide" and refers to polymers of amino acids of any length. These terms also include proteins that are post-translationally modified through reactions that include, but are not limited to, phosphorylation, glycosylation, acetylation, or for eukaryotic host cells also protein processing. Modifications and changes, for example fusions to other proteins, amino acid sequence substitutions, deletions, or insertions, can be made in the structure of a polypeptide while the molecule maintains its biological functional activity. For example, certain amino acid sequence substitutions can be made in a polypeptide or its underlying nucleic acid coding sequence and a protein can be obtained with the same or advantageous properties. The term "polypeptide" typically refers to a sequence of more than 10 amino acids and the term "peptide" means sequences with up to 10 amino acids in length. However, the terms may be used interchangeably.

The term "genomic DNA", or "genome" is used interchangeably and refers to the heritable genetic information of a host organism. For eukaryotic host cells, the genomic DNA comprises the DNA of the nucleus (also referred to as chromosomal DNA) but also of other cellular organelles (e.g., mitochondria).

The term "gene" as used herein refers to a DNA locus of heritable genomic sequence which affects an organism's traits by being expressed as a functional product or by regulation of gene expression. Genes and polynucleotides may include regions that regulate their expression, such as transcription initiation, translation, and transcription termination. Thus, included are regulatory elements such as a promoter.

The term "enhancement", "enhance", "enhanced", "increase" or "increased", as used herein, generally means an increase by at least about 21% as compared to a control cell, for example an increase by at least about 30%, or at least about 40%, or at least about 50%, or at least about 75%, or at least about 80%, or at least about 90%, or at least about 100%, or at least about 200%, or at least about 10-800% as compared to a control cell. As used herein, a "control cell" is for example a wild-type host cell. For example, a "control cell" is a host cell comprising a mutant lacY gene.

In an industrial setting, the term 'lactose killing' typically refers to the phenomenon of growth inhibition or growth arrest of an organism that is grown in an environment in which lactose or galactosidase is not used as the actual carbon source. Typical carbon sources comprise, but are not limited to, glycerol, maltose, glucose, fructose, sucrose, fucose, mannose, sialic acid, starch, cellulose, polyols, such as mannitol, xylitol, sorbitol, organic acids, such as lactate, succinate, acetate, and/or, pentoses, such as xylose and arabinose.

### EXAMPLES

### 1. Material and Methods

### 1.1 Reagents

### Kits:

Plasmid extraction kit (any supplier, we used E.Z.N.A Plasmid DNA Mini Kit I from Omega Bio-Tek)
PCR product purification kit (any supplier, we used Thermo Scientific GeneJET PCR Purification Kit from Thermo Fisher Scientific)
Extraction of DNA fragments from agarose gel (any kit supplier, we used Thermo Scientific GeneJET Gel Extraction Kit from Thermo Fisher Scientific)
DNA-modifying enzymes: restriction endonucleases, T4 ligase, T4 polynucleotide kinase, Antarctic phosphatase, Taq polymerase, Q5 or any other high-fidelity DNA polymerase (we purchased all these enzymes from New England Biolabs)

### Antibiotics:

ampicillin (any supplier, Carl Roth)
kanamycin (any supplier, Carl Roth)
chloramphenicol (any supplier, PanReac AppliChem)

### Electrophoresis buffer components and reagents:

Agarose (PeqGOLD Agarose, universal from VWR)
TRIS base (Carl Roth)
EDTA (Na₂EDTA, Carl Roth)
Acetic acid (100%, Rotipuran, Carl Roth)
Bromophenol blue (Carl Roth)

### Additional general chemicals and materials:

Ethanol (Carl Roth)
Na-acetate (Carl Roth)
Primers (Eurofins Genomics)
media components (suppliers are specified in the media section)
Isopropyl-β-D-thiogalactoside (IPTG) (Gerbu)

### 1.2 Molecular biologic methods

### Standard methods, descriptions can be found in any protocol books:

Plasmid DNA isolation / genomic DNA isolation / DNA fragment isolation were carried out according to the manufacturer's instructions
Enzymatic DNA modifications: restriction digest, ligation, phosphorylation and dephosphorylation of DNA ends were all carried out according to the manufacturer's instructions
PCR (polymerase chain reaction)
Agarose gel electrophoresis
Preparation of electrocompetent *E. coli* cells and transformation by electroporation:

### Genome modification in E. coli:

Deletions and insertions in the E. *coli* genome were carried out with the λ-RED system using plasmids and protocols provided in the publication of Datsenko KA & Wanner BL, Proc Natl Acad Sci U S A. 2000 Jun 6;97(12):6640-5. (doi: 10.1073/pnas.120163297). Plasmids described in the paper were ordered from the Coli Genetic Stock Center at Yale university.

### 1.3 Strains and plasmids

Strains: *(Escherichia coli*)
NEB10-beta (New England Biolabs)
*genotype: Δ(ara-leu)* 7697 *araD139 fhuA ΔlacX74 galK16 galE15e14- ϕ80dlacZΔM15 recA1 relA1 endA1 nupG rpsL (Str^{R}) rph spoT1 Δ(mrr-hsdRMS-mcrBC)*
BL21 (available from multiple suppliers)
genotype: F*⁻ omp*T *hsd*S_{B} (r_{B}⁻, m_{B}⁻) *gal dcm*

### Plasmids (including):

pBluescript II SK (-) (available from multiple suppliers)
pKD46, pDK3, pKD4, pKD13, pCP20 (Datsenko KA & Wanner BL 2000)

### Generating a 2'-FL-producing strain with reduced lactose sensitivity

A genetically engineered derivative of the *E. coli* strain BL21, that was capable of producing the HMO sugar 2'-FL, was prepared. This strain was based on overexpression of the fucose biosynthesis genes *manC, manB, gmd* and *wcag,* as well as overexpression of the α-1,2-fucosyltransferase *futC* from *Helicobacter pylori.* Native level expression of the lactose transporter, *lacY,* represents a bottleneck for efficient 2'-FL production, therefore *lacY* was also overexpressed. By native level of *lacY* expression lactose toxicity occurs only under specific conditions that don't usually occur during a standard fermentation, but overexpression of the *lacY* gene augmented lactose toxicity to a level where it became quite pronounced and strongly influenced growth and cellular health and by causing lysis in a significant proportion of the cells, it also negatively influenced further downstream processing of the material. In order to reduce lactose toxicity in the /acYoverexpressing strain, the above-mentioned point mutations H322R or E325A, or the L293R mutation for reference (from patent WO 2022/136568), were introduced into the lacY expression cassette first in a plasmid by PCR-based site-directed mutagenesis, using synthetic oligonucleotide mismatch primers. From this plasmid template the expression cassettes were amplified by PCR using primers that carried additional 50 base long homology regions to drive genome integration into the *E*. *coli* genome by λ-RED-mediated homologous recombination to selected target sites, according to the publication Datsenko and Wanner 2000. Genome integration in individual clones was confirmed by PCR analysis of the integration site flanks as well as by sequencing the genomic insert. All mutant *lacY* overexpression cassettes were integrated to the same genomic locus, therefore the only difference between all lines are the single respective point mutations in their *lacY* expression cassettes.

### 1.4 Media

### Synthetic medium for shake flask cultures

| | |
|---|---|
| NH₄)₂-H-citrate | 1.00 g/L |
| (NH₄)₂-SO₄ | 2.68 g/L |
| NaH₂PO₄xH₂O | 8.00 g/L |
| K₂HPO₄ | 29.20 g/L |
| Na₂SO₄x10H₂O | 4.54 g/L |
| NH₄Cl | 0.50 g/L |
| LB powder Lennox | 2g/L (all from Carl Roth) |
| Glycerol | 50 g/L (with adjustment to glycerol concentration of the stock: 58 g/L) |
| | (Glycerol 86.5% from Chemsolute/T.H.Geyer) |

### Autoclave then add filter sterilized supplements:

| | |
|---|---|
| MgSO₄ | 0.49 g/L (1:1000 from stock) Carl Roth |
| Thiamine | 0.01 g/L (1:1000 from stock) Carl Roth |
| Trace element solution | 3.0 mL/L (3:1000 from stock) (see below) |

### Trace element solution (TES)

| | | |
|---|---|---|
| CaCl₂·2H₂O | 0.50 g/L | Carl Roth |
| CoCl₂·6H₂O | 0.18 g/L | Carl Roth |
| CuSO₄·5H₂O | 0.16 g/L | Carl Roth |
| FeCl₃·6H₂O | 16.70 g/L | Carl Roth |
| MnSO₄·H₂O | 0.10 g/L | Carl Roth |
| Na₂EDTA·2H₂O | 20.10 g/L | Carl Roth |
| ZnSO₄·7H₂O | 0.18 g/L | Carl Roth |

Lactose was added to the cells upon induction of expression at a concentration of 12.5 g/L from a stock solution of 250 g/L that was sterilized by filtration. (Lactose from Carl Roth)

### 1.5 Cultivation conditions

2'-FL production strains containing the above described lacY-mutant expression cassettes were inoculated in 3 replicates into 25 mL culture media in 250 mL-sized shake flasks to a starting OD₆₀₀ of 0.1 and grown in growth chambers (Labwit ZWYR-D2402) at 30°C with 250 rpm agitation. Once cell density reached an approximate OD₆₀₀ of 0.6 lactose was added to the concentration of 12.5 g/L (36.5 mM) and protein expression was induced. Afterwards the cultures were grown for 72 hours without adding any further materials. Samples were taken at decided time points for following growth by OD600 and for HPLC quantification of the product 2'-FL and residual glycerol and lactose.

### 1.6 Sampling methodology

A sample of 2 mL was taken at any decided time point from the cell suspension and centrifuged at 16,000 g at room temperature. Next the sample was heated to 95 °C for 5 min to precipitate proteins from the solution. All precipitates were then separated by centrifugation at 16,000 g for 5 min at room temperature. To 500 µL of the supernatant 500 µL deionized water was added as well as an additional 500 µL acetonitrile. Afterwards the sample was observed for 30-60 minutes to control if additional precipitation occurs. The sample is then clarified by 5 min centrifugation at 16,000 g at room temperature. Thereafter the sample was filtered using a 2 mL syringe connected to a 0.2 µm syringe filter (Altmann Analytik, PVDF, 13 mm, 0.2 µm, product no.: AG5191-5924) into the HPLC sample vials.

### 1.7 Analytical methods

2'-FL, rest lactose and rest glycerol were quantified by HPLC measurement. We used Agilent 1260 Infinity II MCT, vial sampler, quat pump and 1290 Infinity II ELSD detector. For the separation we used Infinitylab Poroshell 120 HILIC-Z column.

## Claims

1. A mutant galactoside permease comprising the amino acid sequence having at least 97% sequence identity to the amino acid sequence of SEQ ID NO:1 and having at least one mutation, wherein the at least one mutation is at a position that is involved in proton translocation of the (wild-type) galactoside permease according to SEQ ID NO: 1, and wherein the at least one mutation is an amino acid substitution at one or more positions corresponding to positions 322, 325, and/or 302 of the amino acid sequence of SEQ ID NO:1.

2. The mutant galactoside permease according to claim 1, wherein the mutant galactoside permease:
(i) has a reduced proton symporter activity, more preferably a lack of proton symporter activity; and/or
(ii) is not capable of mediating galactoside: proton symport with a 1:1 stoichiometry; and/or
(iii) has a reduced proton translocation activity, preferably a lack of proton translocation activity, as comparted to the (wild-type) galactoside permease according to SEQ ID NO: 1.

3. The mutant galactoside permease according to claim 1 or claim 2, wherein the mutant galactoside permease:
a) is capable of substrate binding and/or has no mutation in the substrate-binding site; and/or
b) has / maintains substrate specificity for galactosides; and/or
c) exhibits / maintains galactoside translocation activity.

4. The mutant galactoside permease according to claim 3, wherein the mutant galactoside permease does not require (full) proton translocation for the binding of galactoside(s).

5. The mutant galactoside permease according to any one of claims 1 to 4, wherein the at least one amino acid substitution comprises a substitution of
(i) a basic amino acid residue against a basic amino acid residue; and/or
(ii) a basic amino acid residue against a polar amino acid residue; and/or
(iii) a basic amino acid residue against a non-polar amino acid residue; and/or
(iv) a basic amino acid residue against an acidic amino acid residue; and/or
(v) an acidic amino acid residue against a non-polar amino acid residue; and/or
(vi) an acidic amino acid residue against a polar amino acid residue; and/or
(vii) an acidic amino acid residue against an acidic amino acid residue; and/or
(viii) an acidic amino acid residue against a basic amino acid residue.

6. The mutant galactoside permease according to any one of claims 1 to 5, wherein:
a) the amino acid substitution at the position corresponding to position 322 of the amino acid sequence of SEQ ID NO: 1 is a substitution of histidine against an amino acid residue selected from any one of Y, Q, C, R, K, N, A, M, F, or D, preferably a substitution of histidine against an amino acid residue selected from any one of Q, C, R, K, or N, more preferably a substitution of histidine against an amino acid residue selected from any one of R or K; and/or
b) the amino acid substitution at the position corresponding to position 325 of the amino acid sequence of SEQ ID NO: 1 is a substitution of glutamic acid against an amino acid residue selected from any one of C, D, A, Q, K, S, or H, preferably a substitution of glutamic acid against an amino acid residue selected from any one of C, D, or A, more preferably a substitution of glutamic acid against an amino acid residue selected from any one of C or A; and/or
c) the amino acid substitution at the position corresponding to position 302 of the amino acid sequence of SEQ ID NO: 1 is a substitution of arginine against an amino acid residue selected from any one of K, H, L, A, S, or C, preferably a substitution of arginine against an amino acid residue selected from any one of K, H, L, A, or S, more preferably a substitution of arginine against an amino acid residue selected from any one of K or H.

7. The mutant galactoside permease according to claim 6, wherein the mutant galactoside permease comprises at least one of the following amino acid substitutions:
(i) H322R at the position corresponding to position 322 of the amino acid sequence of SEQ ID NO: 1; and/or
(ii) E325A at the position corresponding to position 325 of the amino acid sequence of SEQ ID NO: 1; and/or
(iii) R302K at the position corresponding to position 302 of the amino acid sequence of SEQ ID NO: 1.

8. The mutant galactoside permease according to any one of claims 1 to 7, wherein the mutant galactoside permease is a mutant β-galactoside permease, preferably a mutant lactose permease.

9. A nucleic acid sequence encoding the mutant galactoside permease according to any one of claim 1 to 8.

10. A microbial host cell comprising the nucleic acid sequence of claim 9.

11. The microbial host cell of claim 10, wherein the host cell comprises an exogenous nucleic acid sequence encoding a glycosyltransferase, preferably a fucosyltransferase.

12. Use of the mutant galactoside permease according to any one of claims 1 to 8, or the nucleic acid sequence according to claim 9, or the microbial host cell according to claim 10 or claim 11, for the production of (human milk) oligosaccharides.

13. A process for the production of (human milk) oligosaccharide, comprising:
a) culturing the microbial host cell according to claim 10 or claim 11 in the presence of (i) an exogenous carbon source and (ii) a galactoside (sugar), preferably lactose, and under conditions that allow the host cell to produce a (human milk) oligosaccharide, and
b) obtaining the (human milk) oligosaccharide from the culture medium and/or the (non-human) host cell.

14. The process of claim 13, wherein the process is a (fed) batch or a continuous process.

15. The process according to claim 13 or claim 14, wherein the oligosaccharide is selected from the group consisting of 2'-fucosyllactose (2'-FL), 3'-fucosyllactose (3'-FL), 2', 3-difucosyllactose (DFL), lacto-*N*-triose II, lacto-*N*-tetraose (LNT), laclo-*N*-*neo-*tetraose (LNnT), lacto-*N*-fucopentaose I (LNFP-I), lacto-N-neofucopentaose I (LN*n*FP-I), lacto-*N*-fucopentaose II (LNFP-II), lacto-*N*-fucopentaose III (LNFP- III), lacto-*N*-fucopentaose V (LNFP-V), lacto-*N*-neofucopentaose V (LNnFP-V), lacto-*N*-hexaose (LNH), lacto-*N-*neohexaose (LNnH), para-lacto-N-hexaose (paraLNH), para-lacto-N-neohexaose (paraLNnH), difucosyl-lacto-N-neohexaose (DF-LNnH), lacto-N-difucosylhexaose I, lacto-*N*-difucosylhexaose II, *para*-lacto-*N-*fucosylhexaose (paraLNH), fucosyl-lacto-*N-*sialylpentaose a (F-LST-a), fucosyl-lacto-*N*-sialylpentaose b (F-LST-b), fucosyl-lacto-*N*-sialylpentaose c (F-LST-c), disialyl-lacto-*N*-fucopentaose (DS-LNFP), 3-fucosyl-3'-sialyllactose (3F-3'-SL), 3-fucosyl-6'-sialyllactose (3F-6'-SL), lacto-*N-*neodifucohexaose I (LNnDFH I), 3'-sialyllactose (3-SL), 6'-sialyllactose (6-SL), sialyl-lacto-*N*-tetraose a (LST-a), sialyllacto-*N*-tetraose b (LST-b), sialyllacto-*N*-tetraose c (LST-c), disialyllacto-*N*-tetraose (DS-LNT), disialyl-lacto-*N*-fucopentaose V (DS-LNFP V), lacto-N-neodifucohexaose I (LNnDFH I), 3'-galactosyllactose (3'-GL), 6'-galactosyllactose (6'-GL), or derivatives thereof.
